(19) Europäisches Patentamt
European Patent Office
Office européen des brevets

(11) Veröffentlichungsnummer: **0 497 122 A1**

(12) # EUROPÄISCHE PATENTANMELDUNG

(21) Anmeldenummer: **92100393.5**

(22) Anmeldetag: **11.01.92**

(51) Int. Cl.5: **C07D 271/04**, C07D 413/12, A61K 31/41

(30) Priorität: **26.01.91 DE 4102282**

(43) Veröffentlichungstag der Anmeldung:
**05.08.92 Patentblatt 92/32**

(84) Benannte Vertragsstaaten:
**AT BE CH DE DK ES FR GB GR IT LI NL PT SE**

(71) Anmelder: **CASSELLA Aktiengesellschaft**
**Hanauer Landstrasse 526**
**W-6000 Frankfurt am Main 60(DE)**

(72) Erfinder: **Schönafinger, Karl, Dr.**
**Holunderweg 8**
**W-8755 Alzenau(DE)**
Erfinder: **Binder, Rudolf, Dr.**
**Markgrafenstrasse 65**
**W-8000 München 82(DE)**
Erfinder: **Bohn, Helmut, Dr.**
**Kranzbergring 11**
**W-6369 Schöneck 1(DE)**
Erfinder: **Just, Melitta, Dr.**
**Theodor-Heuss-Strasse 80**
**W-6070 Langen 2(DE)**

(74) Vertreter: **Urbach, Hans-Georg, Dr. et al**
**CASSELLA AKTIENGESELLSCHAFT**
**Patentabteilung Hanauer Landstrasse 526**
**W-6000 Frankfurt am Main 60(DE)**

(54) **Substituierte 3-Aminosydnonimine, Verfahren zu ihrer Herstellung und ihre Verwendung.**

(57) Die vorliegende Erfindung betrifft 3-Aminosydnonimine der allgemeinen Formel I

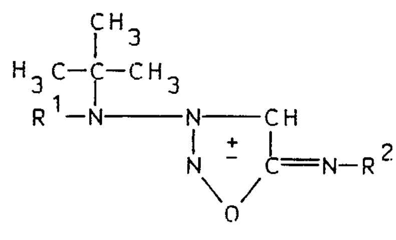

(I)

und ihre pharmakologisch annehmbaren Säureadditionssalze, worin $R^1$ und $R^2$ wie in der Beschreibung angegeben definiert sind, Verfahren zu ihrer Herstellung und ihre Verwendung.



Rank Xerox (UK) Business Services

Die vorliegende Erfindung betrifft pharmakologisch wirksame substituierte 3-Aminosydnonimine, Verfahren zu ihrer Herstellung und ihre Verwendung.

3-Aminosydnonimine sind bereits aus einer Reihe von Veröffentlichungen bekannt. Den erfindungsgemäßen Verbindungen strukturell verwandt sind die in der EP-A-59356 und insbesondere in der EP-A-346684 offenbarten.

Die Erfindung betrifft 3-Aminosydnonimine der allgemeinen Formel I

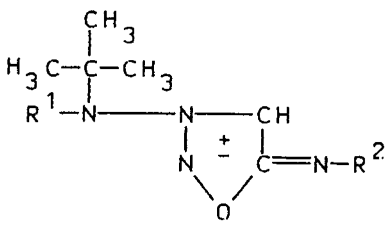

(I)

und ihre pharmakologisch annehmbaren Säureadditionssalze,
worin

$R^1$ Alkyl mit 1 bis 6 C-Atomen;

$R^2$ Wasserstoff oder den Rest $-COR^3$; und

$R^3$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen; oder einen fünf- oder sechsgliedrigen Heterocyclus, der 1 bis 3 Stickstoff-, Sauerstoff- und/oder Schwefelatome als Ringglieder aufweisen und auch benzanelliert sein kann, bedeutet.

Aliphatische Reste, Alkylreste und Alkoxyreste können geradkettig oder verzweigt sein. Dies gilt auch, wenn sie als Substituenten anderer Reste, z.B. als Substituenten für Arylreste, oder in Verbindung mit anderen Resten auftreten, z.B. als Alkoxycarbonyl.

$R^1$ steht bevorzugt für Alkyl mit 1 bis 4 C-Atomen und bedeutet besonders bevorzugt Methyl, Ethyl, n-Propyl oder n-Butyl.

Als für $R^3$ stehende aliphatische Reste kommen insbesondere Alkylreste in Betracht. Als für $R^3$ stehende aliphatische Reste, die durch Alkoxy mit 1 bis 3 C-Atomen substituiert sind, ist insbesondere der Methoxymethylrest zu nennen. Als für $R^3$ stehende cycloaliphatische Reste kommen vor allem Cycloalkylreste mit 5 bis 7 C-Atomen, insbesondere Cyclopentyl, und bevorzugt Cyclohexyl, in Betracht. Als für $R^3$ stehender bicycloaliphatischer Rest kommt insbesondere das 2,6,6-Trimethylbicyclo [3.1.1] heptan-3-yl (=Pinanyl-3) in Betracht.

Als für $R^3$ stehender tricycloaliphatischer Rest kommt insbesondere das Tricyclo [$3.3.1.1^{3,7}$] decan-1-yl (=Adamantanyl) in Betracht. Als für $R^3$ stehende Alkoxyreste kommen insbesondere solche mit 1 bis 4 C-Atomen in Betracht.

Als für $R^3$ stehende Alkoxycarbonylreste kommen insbesondere solche mit insgesamt 2 bis 4 C-Atomen, vor allem der Ethoxycarbonylrest in Betracht. Als für $R^3$ stehende Arylreste sind z.B. $\alpha$- oder $\beta$-Naphthylreste und der Phenylrest, zu nennen. Als für $R^3$ stehende Aryloxyreste sind z.B. $\alpha$-oder $\beta$-Naphthoxyreste und der Phenoxyrest, zu nennen. Die für $R^3$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Als Halogensubstituenten für die Arylreste kommen z.B. Chlor und Bromatome in Betracht. Als für $R^3$ stehende substituierte Arylreste sind insbesondere zu nennen: Methylphenyl (= Tolyl), Methoxyphenyl, Nitrophenyl und Chlorophenyl. Fünf- oder sechsgliedrige Heterocyclen, die für $R^3$ stehen können, sind beispielsweise Thiophen, Di- oder Tetrahydrothiophen, Pyrrol, Pyrrolin, Pyrrolidin, Pyridin, Dihydropyridin, Piperidin, Pyran, Perhydropyran, Imidazol, Imidazolin, Imidazolidin, Oxazol, Oxazolin, Oxazolidin, Thiazol, Thiazolin, Thiazolidin, Pyrimidin, Pyridazin, Pyrazin, Piperazin, Morpholin und Thiomorpholin. Ein benzanellierter Heterocyclus ist insbesondere Indol.

Bevorzugte Reste $R^3$ sind Alkyl mit 1 bis 4 C-Atomen; Alkoxy mit 1 bis 4 C-Atomen; Phenyl; mit Halogen und/oder Nitro oder Alkoxy mit 1 bis 3 C-Atomen substituiertes Phenyl und Pyridyl.

Besonders bevorzugte Reste $R^3$ sind Methyl, Ethyl, n-Propyl, Isopropyl, tert.-Butyl, Methoxy, Ethoxy, Phenyl, 2-Nitrophenyl, 4-Nitrophenyl, 4-Chlorphenyl, 4-Methoxyphenyl, 4-Ethoxyphenyl und 3-Pyridyl.

Eine Verbindung der allgemeinen Formel I kann dadurch hergestellt werden, daß eine Verbindung der allgemeinen Formel II

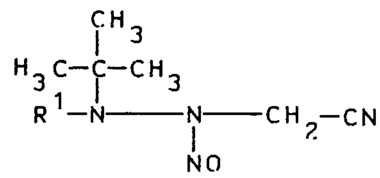

(II)

worin $R^1$ die oben genannte Bedeutung besitzt, zu einer Verbindung der allgemeinen Formel Ia

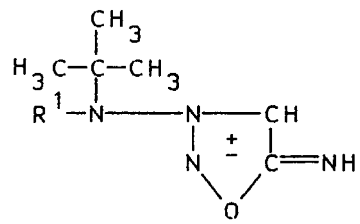

(Ia)

cyclisiert wird und daß diese oder ein Säureadditionssalz davon für den Fall, daß eine Verbindung der Formel I mit $R^2$ = $-COR^3$ hergestellt werden soll, mit einem Acylierungsmittel, das den Rest $-COR^3$ einführt, acyliert wird und die so erhaltene Verbindung gegebenenfalls in ein pharmakologisch annehmbares Säureadditionssalz überführt wird.

Die Cyclisierung der Verbindungen II zu den Verbindungen Ia wird in einem geeigneten organischen oder anorganischen Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert unter 3 einstellen, also z.B. starke Säuren, wie Mineralsäuren, wie Schwefel-, Salpeter- oder Phosphorsäure, vorzugsweise Chlorwasserstoff, aber auch starke organische Säuren, wie Trifluoressigsäure. Die Cyclisierung wird normalerweise unter Eiskühlung durchgeführt. Von dem Cyclisierungsmittel kommt z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Di-butylether, Tetrahydrofuran, 1,4-Dioxan, 1,2-Dimethoxyethan, Bis-$\beta$-methoxyethylether; Oligoethylen-glykol-dimethylether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl- oder -isobutyl-ester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butyl-ester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig-und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid,

Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugsweise Essigsäureethylester-Methanol.

Die Verbindungen der Formel Ia stellen erfindungsgemäße Verbindungen der allgemeinen Formel I für den Fall dar, daß $R^2$ = Wasserstoff ist.

Die Acylierung der Verbindung der Formel Ia, die auch in Form eines Säureadditionssalzes vorliegen kann, zur Einführung des Restes $R^2$ = $-COR^3$ kann in an sich bekannter Weise mit einem geeigneten Acylierungsmittel der Formel III durchgeführt werden

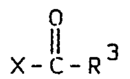

(III)

worin X ein nukleophil abspaltbarer Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; $-O-CO-R^3$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird zweckmäßigerweise in flüssiger Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel III 1 : 1. Zweckmäßigerweise wird das Acylierungsmittel der Formel III in einem geringen molaren Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1: (1 bis 1,2). Falls bei der Acylierungsreaktion eine Säure abgespalten wird, ist der Zusatz eines Säurefängers, wie z.B. eines Alkalihydroxids, wie z.B. Natrium-, Kalium- oder Lithium-hydroxid, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prinzipiell bei Temperaturen zwischen -10°C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar: für X = Halogen Säurehalogenide bzw. Halogenameisensäureester, von denen Säurechloride und Chlorameisensäureester bevorzugt sind; für -OH Carbonsäuren; für -O-Alkyl und -O-Aryl Ester, von denen die Tolyl-, 2,4-Dinitro- oder 4-Nitrophenylester bevorzugt sind; für $-O-CO-R^1$ Anhydride; für -O-CO-O-Alkyl gemischte Carbonsäure-Kohlensäure-Anhydride; oder heterocyclische Amide oder Azolide, insbesondere von N,N'-Carbonyldiazolen, wie z.B. N,N'-Carbonyldiimidazol, 2,2'-Carbonyl-ditriazol(1,2,3), 1,1'-Carbonyl-ditriazol-(1,2,4), N,N'-Carbonyl-dipyrazol, 2,2'-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N'-disubstituierte Carbodiimide, insbesondere wenn sie mindestens einen sekundären oder tertiären Alkylrest enthalten, wie z.B. Diisopropyl-, Dicyclohexyl- oder N-Methyl-N'-tert.butylcarbodiimid (vgl. Methodicum Chimicum, Verlag G.Thieme, Stuttgart, Bd.6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z B. N,N'-Disuccinimido-carbonat, Diphthalimido-carbonat, 1,1'-(Carbonyldioxy)-dibenzo-triazol oder Di-2-pyridyl-carbonat (vgl. z.B. Tetrahedron Letters, Vol.25, No.43, 4943-4946), gegebenenfalls in Anwesenheit geeigneter Katalysatoren, wie z.B. 4-Dimethylaminopyridin. Ferner sind als Aktivierungsmittel N,N'-Carbonyldiazole, wie z.B. N,N'-Carbonyl-diimidazol, 2,2'-Carbonyl-ditriazol(1,2,3), 1,1'-Carbonyl-ditriazol(1,2,4), N,N'-Carbonyl-dipyrazol, 2,2'-Carbonyl-ditetrazol, N,N'-Carbonyl-di-benzimidazol oder N,N'-Carbonyl-dibenztriazol, geeignet (vgl. z.B. H.A. Staab, M. Lücking und F.H. Dürr, loc. cit; H.A. Staab und A. Mannschreck loc. cit.; H.A. Staab und W. Rohr loc. cit). Als N,N'-Carbonyl-diazol wird häufig das käufliche N,N'-Carbonyldiimidazol verwendet. Die anderen N,N'-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-PS 2 139 225) oder N,N'-Oxalyl-diazole wie z.B. 1,1'-Oxalyldi-imidazol, 1,1'-Oxalyldi-1,2,4-triazol und 1,1'-Oxalyldi-1,2,3,4-tetrazol (vgl. z.B. Shizuaka Murata, Bull.Chem. Soc.Jap. 57, 3597-3598 (1984)); Methylethylphosphinsäureanhydrid (vgl. z.B. DE-OS 31 01 427); Diphosphortetrajodid (Chem. Lett. 1983, 449); Dialkyldisulfit (Indian J. Chem. 21, 259 (1982)); oder andere reaktive Agentien.

Geeignete Lösungs-, Dispergier- oder Verdünnungsmittel sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die substituierten 3-Amino-sydnonimine der allgemeinen Formel I bilden mit anorganischen oder organischen Säuren Säureadditionssalze. Zur Bildung derartiger Säureadditionssalze sind anorganische oder organische Säuren geeignet. Geeignete Säuren sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden.

Bei der Synthese der Verbindungen der Formel Ia fallen normalerweise die Säureadditionssalze an. Aus den Säureadditionssalzen können die freien Verbindungen der allgemeinen Formel I bzw. Ia gewünschtenfalls in bekannter Weise, d.h. durch Auflösen oder Suspendieren in Wasser und Alkalischstellen, z.B. mit Natronlauge, und anschließendes Isolieren, gewonnen werden.

Die benötigten Ausgangsverbindungen der allgemeinen Formel II können in an sich bekannter Weise nach der Strecker'schen Aminonitrilsynthese aus Verbindungen der allgemeinen Formel IV

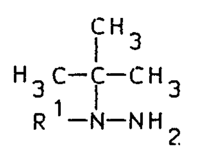

(IV)

worin $R^1$ die oben genannte Bedeutung besitzt, durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel V

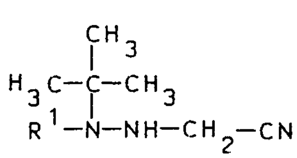

(V)

entsteht, die durch Nitrosierung in die Verbindung II überführt wird. Die Nitrosierung wird in bekannter Weise in einem geeigneten Lösungsmittel, vorzugsweise in Wasser, z.B. bei Temperaturen von 0 bis 10°C durchgeführt. Die salpetrige Säure wird dabei normalerweise aus einem

Alkalimetallnitrit, z.B. Natriumnitrit, und Salzsäure erzeugt. Es ist zweckmäßig, die wäßrige Lösung der Verbindung V mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten

organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel IV sind zum Teil bekannt bzw. lassen sich, ausgehend von Verbindungen der allgemeinen Formel VI

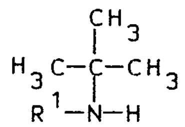

(VI)

dadurch herstellen, daß entweder

a) eine Verbindung der Formel VI zur N-Nitrosoverbindung VII nitrosiert und anschließend, zweckmäßigerweise mit Lithiumaluminiumhydrid, reduziert wird:

(VI)

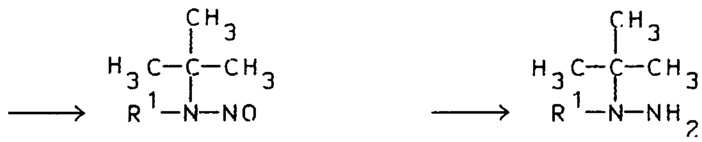

(VII) (IV)

oder in an sich bekannter Weise

b) eine Verbindung der Formel VI mit Kaliumcyanat im sauren Medium in das Harnstoffderivat VIII überführt wird, das dann durch Oxydation mit Natriumhypochlorit nach dem Hoffmann-Abbau in die Verbindung IV überführt wird.

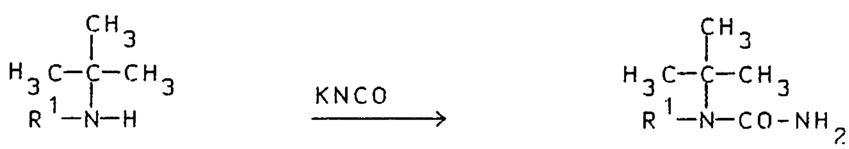


(VI) (VIII)

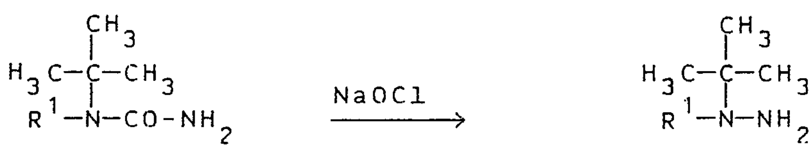


(VIII) (IV)

Die Herstellung der Ausgangsverbindungen der Formeln IV und VI ist bekannt oder kann nach an sich bekannten Verfahren durchgeführt werden.

Die Verbindungen der allgemeinen Formel I und ihre pharmakologisch annehmbaren Säureadditionssalze besitzen wertvolle pharmakologische Eigenschaften. Besonders ausgeprägt ist ihre Wirkung auf das Herz-Kreislauf-System. Verglichen mit bekannten, in 3-Stellung substituierten Sydnoniminverbindungen, z.B. solchen der EP-A-59356 oder EP-A-346684, sowie der im Handel befindlichen strukturähnlichen Verbindung Molsidomin, besitzen sie überraschenderweise eine wesentlich längere Wirkungsdauer. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Durch Hemmung der Thrombocytenaggregation können die Verbindungen außerdem antithrombotische Effekte zeigen.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionsssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der Verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). In der folgenden Tabelle sind so erhaltene $IC_{50}$-Werte angegeben. Wie der Vergleich mit den $IC_{50}$-Wert für bekannte Verbindungen aus dem Stand der Technik ergibt, sind die erfindungsgemäßen Verbindungen wirksamer.

| Verbindung | $IC_{50}$ |
|---|---|
| Beispiel 1<br>Beispiel 6<br>N-Ethoxycarbonyl-3-morpholinosydnonimin (Molsidomin)<br>3-(tert.Butyl-(2-diisopropylaminoethyl)-amino)-sydnonimin-dihydrochlorid<br>(Beispiel 6 aus EP-A-346684) | $3{,}0.10^{-6}$<br>$3{,}0.10^{-6}$<br>$3{,}0.10^{-4}$<br>$4{,}0.\ 10^{-6}$ |

<u>Beispiel 1</u>

3-(N-tert.-Butyl-N-methylamino)sydnonimin-hydrochlorid

a) Zur Mischung von 6.1 g

N-tert.-Butyl-N-methylhydrazinoacetonitril, 25 ml Wasser, 25 ml Essigsäureethylester und 4 ml 10 N Salzsäure wird eine Lösung von 4.5 g Natriumnitrit in 20 ml Wasser getropft. Nach 4 Stunden Rühren bei Raumtemperatur wird die organische Phase abgetrennt, die wässrige Phase noch zweimal mit wenig Essigsäureethylester ausgeschüttelt. Die vereinigten organischen Phasen werden mit 20 ml einer isopropanolischen Salzsäurelösung versetzt, 15 Stunden bei Raumtemperatur nachgerührt und im Wasserstrahlvakuum eingeengt. Der Rückstand wird auf einer Kieselgelsäule mit einer 9:1-Mischung von Methylenchlorid und Methanol chromatographiert und der Rückstand, der nach Einengen der Fraktion, die die gewünschte Verbindung enthält-, zurückbleibt, nochmals mit Essigsäureethylester aufgekocht, abgesaugt und getrocknet.
Ausbeute: 0.50 g
Schmelzpunkt: 148° (Zers.)

b) Das unter a) benötigte Ausgangsmaterial

N-tert.-Butyl-N-methylhydrazinoacetonitril wird auf folgende Weise hergestellt:
Zur eisgekühlten Mischung von 22.6 g
N-tert.-Butyl-N-methylhydrazin, 22.2 ml 10 N Salzsaure und 120 ml Wasser wird eine Lösung von 15,8 g Kaliumcyanid in 50 ml Wasser getropft und der pH-Wert mit Salzsäure auf 7 eingestellt. Dann werden 17.0 g einer 39 %igen Formaldehydlösung zugesetzt, der pH-Abfall durch Zugabe einer Sodalösung kompensiert und die Reaktionsmischung 2 Stunden bei 0°C und weitere 3 Stunden bei Raumtemperatur gerührt. Das Produkt wird mit Essigsäureethylester extrahiert, die organische Phase mit verdünntem Eisessig gewaschen und über Natriumsulfat getrocknet. Nach dem Einengen verbleibt ein öliger Rückstand (13.0 g), der ohne weitere Reinigung gemäß a) weiterverarbeitet wird.

<u>Beispiel 2</u>

3-(N-tert.-Butyl-N-propylamino)sydnonimin-hydrochlorid

a) Zur Mischung von 19.5 g

N-tert.-Butyl-N-propylhydrazinoacetonitril, 60 ml Wasser, 60 ml Essigsäureethylester und 12 ml 10 N Salzsäure wird eine Lösung von 11.9 g Natriumnitrit in 40 ml Wasser getropft. Nach 4 Stunden Rühren bei Raumtemperatur wird die organische Phase abgetrennt, die wässrige Phase noch zweimal mit wenig Essigsäureethylester ausgeschüttelt. Die vereinigten organischen Phasen werden mit 42 ml einer isopropanolischen Salzsäurelösung versetzt, 40 Stunden bei Raumtemperatur nachgerührt und im Wasserstrahlvakuum eingeengt. Der Rückstand wird mit Essigsäureethylester aufgekocht, abgesaugt und getrocknet.
Ausbeute: 5.40 g
Schmelzpunkt 143° (Zers.)

b) Das unter a) benötigte Ausgangsmaterial

N-tert.-Butyl-n-propylhydrazinoacetonitril wird auf folgende Weise hergestellt:
Zur eisgekühlten Mischung von 36.3 g

N-tert.-Butyl-N-propylhydrazin, 28.0 ml 10 N Salzsäure und 170 ml Wasser wird eine Lösung von 20.0 g Kaliumcyanid in 70 ml Wasser getropft und der pH-Wert mit Salzsäure auf 7 eingestellt. Dann werden 21.5 g einer 39 %igen Formaldehydlösung zugesetzt, der pH-Abfall durch Zugabe einer Sodalösung kompensiert und die Reaktionsmischung 2 Stunden bei 0°C und weitere 20 Stunden bei Raumtemperatur gerührt. Das Produkt wird mit Essigsäureethylester extrahiert, die organische Phase mit verdünntem Eisessig gewaschen und über Natriumsulfat getrocknet. Nach dem Einengen verbleibt ein öliger Rückstand (39.2 g), der ohne weitere Reinigung gemäß a) weiterverarbeitet wird.

Beispiel 3

3-(N-tert.-Butyl-N-propylamino)-N-(2,2-dimethyl-propionyl)-sydnonimin

2.8 g nach Beispiels 2a) hergestelltes 3-(N-tert.-Butyl-N-propylamino)sydnonimin-hydrochlorid werden in 40 g Wasser gelöst, bei 0° mit 2.5 g Natriumhydrogencarbonat und nach fünfminütigem Rühren mit einer Lösung von 1.6 g Pivalinsäurechlorid in 40 ml Essigsäureester versetzt. Das Reaktionsgemisch wird 5 Stunden lang bei Raumtemperatur gerührt, dann werden die Phasen getrennt, die wäßrige Phase noch einmal mit Essigsäureethylester ausgeschüttelt und die vereinigten organischen Phasen getrocknet und die flüchtigen Bestandteile i. Vak. abgezogen. Der Rückstand wird aus Benzin umkristallisiert.
Ausbeute: 0.95 g
Schmelzpunkt: 96-98° (Zers.)

Beispiel 4

3-(N-tert.-Butyl-N-propylamino)-N-(nicotinoyl)sydnonimin

2.8 g nach Beispiel 2a) hergestelltes 3-(N-tert.-Butyl-N-propylamino)sydnonimin-hydrochlorid werden unter Rührung und Außenkühlung in eine Lösung von 3.4 g Nicotinsäurechlorid in 30 ml Pyridin eingetragen. Nach 16 Stunden Rühren bei Raumtemperatur werden die flüchtigen Bestandteile i. Vak. abgezogen, der Rückstand mit Wasser und Methylenchlorid aufgenommen, die wässrige Phase zweimal mit Methylenchlorid ausgeschüttelt und die vereinigten organischen Phasen nach Trocknen i. Vak. eingeengt. Der Rückstand wird aus Benzin umkristallisiert.
Ausbeute: 1.30 g
Schmelzpunkt: 93-95°

Beispiel 5

3-(N-tert.-Butyl-N-propylamino)-N-(4-methoxy-benzoyl)sydnonimin

2.8 g nach Beispiel 2a) hergestelltes 3-(N-tert.-Butyl-N-propylamino)sydnonimin-hydrochlorid werden in 40 g Wasser gelöst, bei 0° mit 2,5 g Natriumhydrogencarbonat und nach fünfminütigem Rühren mit einer Lösung von 2.25 g Anissäurechlorid in 40 ml Essigsäureethylester versetzt. Das Reaktionsgemisch wird 5 Stunden lang bei Raumtemperatur gerührt, dann werden die Phasen getrennt, die wässrige Phase noch einmal mit Essigsäureethylester ausgeschüttelt und die vereinigten Phasen getrocknet und die flüchtigen Bestandteile i. Vak. abgezogen. Der Rückstand kristallisiert beim Verrühren mit Diethylether, er wird abgesaugt, mit Ether gewaschen und getrocknet.
Ausbeute: 1.80 g
Schmelzpunkt: 90-92°

Beispiel 6

3-(N-tert.-Butyl-N-butylamino)sydnonimin-hydrochlorid

a) Zu einer Lösung von 66.7 g

N-tert.-Butyl-N-n-butylhydrazinoacetonitril in 75 ml Wasser und 33 ml konz. Salzsäure wird 0-5°C 27.62 g Natriumnitrit zugegeben. Es wird 2 Stunden lang bei Raumtemperatur nachgerührt, das Reaktionsgemisch

mit Methylenchlorid extrahiert, die Methylenchloridphase getrocknet und i. Vak. vom Lösungsmittel befreit. Der ölige Rückstand (ca. 60 g) wird unter Eiskühlung mit 140 ml einer ca. 10 %igen Lösung von Chlorwasserstoff in Essigsäureethylester versetzt und 20 Stunden bei 0 bis 5°C stehengelassen. Der entstandene Niederschlag wird abgesaugt, auf einer Kieselgelsäule mit einer 95:5-90:10-Mischung von Methylenchlorid und Methanol chromatographiert und die Fraktion, die die gewünschte Verbindung enthält, eingeengt.
Ausbeute: 12.41 g
Schmelzpunkt: 129-133° (Zers.)

b) Das unter a) benötigte Ausgangsmaterial

N-tert.-Butyl-N-n-butylhydrazinoacetonitril wird auf folgende Weise hergestellt:
Zur eisgekühlten Mischung von 53.0 g N-tert.-Butyl-N-n-butylhydrazin, 32.0 ml konz. Salzsäure und 100 ml Wasser werden 21.6 g Natriumcyanid zugesetzt und der pH-Wert mit Salzsäure auf 7.5 eingestellt. Dann werden 33.9 g einer 39 %igen Formaldehydlösung zugetropft, der pH mit einer 15 %igen Sodalösung wieder auf ca. 7.5 eingestellt und die Reaktiosnmischung 24 Stunden gerührt. Dann werden weitere 4.32 g Natriumcyanid und 6.78 g Formaldehydlösung zugegeben und nochmals 8 Stunden lang bei Raumtemperatur gerührt. Die Lösung wird mit Methylenchlorid extrahiert, die Methylenchloridphase getrocknet und i. Vak. vom Lösungsmittel befreit. Nach dem Einengen verbleibt ein öliger Rückstand (67.2 g), der ohne weitere Reinigung gemäß a) weiterverarbeitet wird.

Beispiel 7

3-(N-tert.-Butyl-N-n-butylamino)-N-(ethoxycarbonyl)sydnonimin

3.0 g 3-(N-tert.-Butyl-N-n-butylamino)sydnoniminhydrochlorid werden in 30 ml Wasser gelöst und mit 2.77 g Natriumhydrogencarbonat versetzt. Zu dieser Lösung tropft man eine Lösung von 1.63 g Chlorameisensäureethylester in 30 ml Methylenchlorid zu. Nach 1 1/2-stündigem Rühren bei Raumtemperatur wird die organische Phase abgetrennt, die wäßrige Phase mehrmals mit Methylenchlorid extrahiert, die vereinigten organischen Phasen getrocknet und im Wasserstrahlvakuum vom Lösungsmittel befreit. Der Rückstand wird auf einer Kieselgelsäule mit einer 98:2-Mischung von Methylenchlorid und Methanol chromatographiert und die Fraktion, die die gewünschte Verbindung enthält, eingeengt.
Ausbeute: 3.60 g
Schmelzpunkt: - (Öl)

Beispiel 8

3-(N-tert.-Butyl-N-methylamino)-N-(benzoyl)sydnonimin

wie in Beispiel 7 beschrieben, unter Verwendung von 2.12g Benzoylchlorid an Stelle des Chlorameisensäureethylesters.
Ausbeute: 1.50 g
Schmelzpunkt: - (Öl)
In den nachfolgenden Beispielen A bis F werden pharmazeutische Präparate beschrieben.

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | pro ml |
|---|---|
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

Emulsion, enthaltend 3 mg Wirkstoff Pro 5 ml:

| | pro 100 ml Emulsion |
|---|---|
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

Rektale Arzneiform, enthaltend 4 mg Wirkstoff pro Suppositorium

| | pro Suppositorium |
|---|---|
| Wirkstoff | 4 mg |
| Suppositoriengrundmasse | ad 2 g |

Beispiel E

Tabletten, enthaltend 2 mg Wirkstoff pro Tablette

| | pro Tablette |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel F

Dragees, enthaltend 1 mg Wirkstoff pro Dragee

| | pro Dragee |
|---|---|
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

## Patentansprüche

**1.** 3-Aminosydnonimine der allgemeinen Formel I

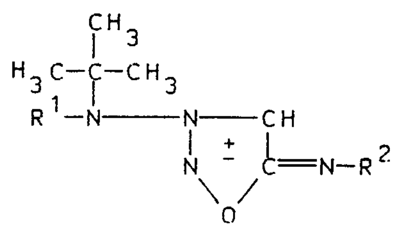

(I)

und ihre pharmakologisch annehmbaren Säureadditionssalze,
worin
$R^1$ Alkyl mit 1 bis 6 C-Atomen;
$R^2$ Wasserstoff oder den Rest $-COR^3$; und
$R^3$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen; oder einen fünf- oder sechsgliedrigen Heterocyclus, der 1 bis 3 Stickstoff-, Sauerstoff- und/oder Schwefelatome als Ringglieder aufweisen und auch benzanelliert sein kann,
bedeutet.

**2.** 3-Aminosydnonimine gemäß Anspruch 1, dadurch gekennzeichnet, daß $R^1$ Methyl, Ethyl, n-Propyl oder n-Butyl bedeutet.

**3.** 3-Aminosydnonimine gemäß Anspruch 1 und/oder 2, dadurch gekennzeichnet, daß $R^3$ Alkyl mit 1 bis 4 C-Atomen; Alkoxy mit 1 bis 4 C-Atomen; Phenyl; mit Halogen und/oder Nitro oder Alkoxy mit 1 bis 3 C-Atomen substituiertes Phenyl oder Pyridyl bedeutet.

**4.** 3-Aminosydnonimine gemäß einem oder mehreren der Ansprüche 1 bis 3, dadurch gekennzeichnet, daß $R^3$ Methyl, Ethyl, n-Propyl, Isopropyl, tert.-Butyl, Methoxy, Ethoxy, Phenyl, 2-Nitrophenyl, 4-Nitrophenyl, 4-Chlorphenyl, 4-Methoxyphenyl, 4-Ethoxyphenyl oder 3-Pyridyl bedeutet.

**5.** Verfahren zur Herstellung der in einem oder mehreren der Ansprüche 1 bis 4 angegebenen Verbindungen der allgemeinen Formel I, dadurch gekennzeichnet, daß eine Verbindung der allgemeinen Formel II

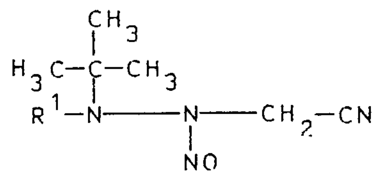

( II )

worin $R^1$ die in Anspruch 1 und/oder 2 angegebenen Bedeutungen besitzt, zu einer Verbindung der Formel Ia

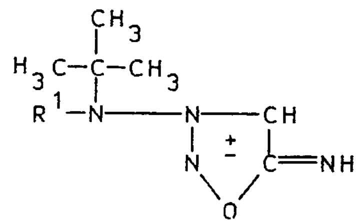

( Ia )

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit $R^2$ = $-COR^3$ hergestellt wird, die Verbindung der Formel Ia oder ein Säureadditionssalz davon mit einem Acylierungsmittel acyliert wird, das den Rest $-COR^3$ einführt und die erhaltene Verbindung gegebenenfalls in ein Säureadditionssalz überführt wird.

**6.** Verfahren nach Anspruch 5, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

**7.** Verwendung von 3-Aminosydnoniminen der allgemeinen Formel I gemäß einem oder mehreren der Ansprüche 1 bis 4 oder pharmakologisch annehmbare Säureadditionssalze davon zur Herstellung von Arzneimitteln zur Bekämpfung und Vorbeugung cardiovaskulärer Erkrankungen.

**8.** Pharmazeutisches Präparat, dadurch gekennzeichnet, daß es ein 3-Aminosydnonimin der allgemeinen Formel I gemäß einem oder mehreren der Ansprüche 1 bis 4 oder ein pharmakologisch annehmbares Säureadditonssalz davon als Wirkstoff zusammen mit pharmazeutisch annehmbaren Träger- und Zusatzstoffen und gegebenenfalls noch ein oder mehrere andere pharmakologische Wirkstoffe enthält.

**Patentansprüche für folgende Vertragsstaaten : ES, GR**

**1.** Verfahren zur Herstellung von 3-Aminosydnoniminen der allgemeinen Formel I

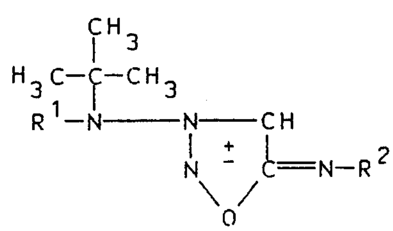

( I )

und ihre pharmakologisch annehmbaren Säureadditionssalze,
worin
$R^1$ Alkyl mit 1 bis 6 C-Atomen;
$R^2$ Wasserstoff oder den Rest $-COR^3$; und
$R^3$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen

Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen; oder einen fünf- oder sechsgliedrigen Heterocyclus, der 1 bis 3 Stickstoff-, Sauerstoff- und/oder Schwefelatome als Ringglieder aufweisen und auch benzanelliert sein kann,
bedeutet,
dadurch gekennzeichnet, daß eine Verbindung der allgemeinen Formel II

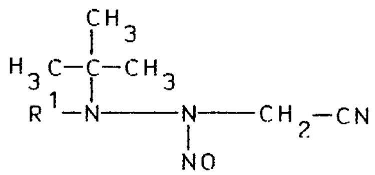

(II)

worin $R^1$ die oben angegebene Bedeutung besitzt, zu einer Verbindung der Formel Ia

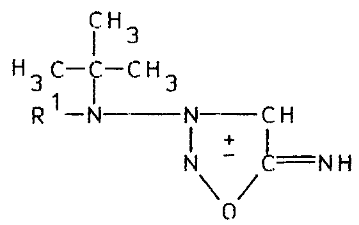

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit $R^2$ = $-COR^3$ hergestellt wird, die Verbindung der Formel Ia oder ein Säureadditionssalz davon mit einem Acylierungsmittel acyliert wird, das den Rest $-COR^3$ einführt und die erhaltene Verbindung gegebenenfalls in ein Säureadditionssalz überführt wird.

2. Verfahren nach Anspruch 1, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

3. Verfahren gemäß Anspruch 1 und/oder 2, dadurch gekennzeichnet, daß $R^1$ Methyl, Ethyl, n-Propyl oder n-Butyl bedeutet.

4. Verfahren gemäß einem oder mehreren der Ansprüche 1 bis 3, dadurch gekennzeichnet, daß $R^3$ Alkyl mit 1 bis 4 C-Atomen; Alkoxy mit 1 bis 4 C-Atomen; Phenyl; mit Halogen und/oder Nitro oder Alkoxy mit 1 bis 3 C-Atomen substituiertes Phenyl oder Pyridyl bedeutet.

5. Verfahren gemäß einem oder mehreren der Ansprüche 1 bis 4, dadurch gekennzeichnet, daß $R^3$ Methyl, Ethyl, n-Propyl, Isopropyl, tert.-Butyl, Methoxy, Ethoxy, Phenyl, 2-Nitrophenyl, 4-Nitrophenyl, 4-Chlorphenyl, 4-Methoxyphenyl, 4-Ethoxyphenyl oder 3-Pyridyl bedeutet.

6. Verwendung von 3-Aminosydnoniminen der allgemeinen Formel I gemäß einem oder mehreren der Ansprüche 1 bis 5 oder pharmakologisch annehmbare Säureadditionssalze davon zur Herstellung von Arzneimitteln zur Herstellung von Arzneimitteln zur Bekämpfung und Vorbeugung cardiovaskulärer Erkrankungen.

7. Verfahren zur Herstellung eines pharmazeutischen Präparates enthaltend ein 3-Aminosydnonimin der allgemeinen Formel I gemäß einem oder mehreren der Ansprüche 1 bis 5 oder ein pharmakologisch annehmbares Säureadditionssalz davon, dadurch gekennzeichnet, daß man dieses zusammen mit

pharmazeutisch annehmbaren Träger-und Zusatzstoffen und gegebenenfalls noch einem oder mehreren anderen pharmakologischen Wirkstoffen in eine geeignete Darreichungsform bringt.

Europäisches Patentamt

# EUROPÄISCHER RECHERCHENBERICHT

Nummer der Anmeldung

EP 92 10 0393

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | EP-A-0 364 684 (CASSELLA AKTIENGESELLSCHAFT)<br>* das ganze Dokument *<br>--- | 1-8 | C07D271/04<br>C07D413/12<br>A61K31/41 |
| X | EP-A-0 367 036 (CASSELLA AKTIENGESELLSCHAFT)<br>* das ganze Dokument, insbesondere Seite 8, Zeilen 32-39, Beispiel 2 in Zusammenhang mit Beispiel 1, und Anspruch 11* | 1-8 | |
| Y | * das ganze Dokument *<br>--- | 1-8 | |
| Y | US-A-3 312 690 (K. MASUDA)<br>* das ganze Dokument *<br>--- | 1-8 | |
| Y | US-A-3 769 283 (K. MASUDA)<br>* das ganze Dokument *<br>----- | 1-8 | |

| RECHERCHIERTE SACHGEBIETE (Int. Cl.5 ) |
|---|
| C07D |

Der vorliegende Recherchenbericht wurde für alle Patentansprüche erstellt

| Recherchenort | Abschlußdatum der Recherche | Prüfer |
|---|---|---|
| DEN HAAG | 29 APRIL 1992 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)